# EUROPÄISCHE PATENTSCHRIFT

(11) **EP 1 908 703 B1**
(45) Veröffentlichungstag und Bekanntmachung des Hinweises auf die Patenterteilung: **08.06.2011**
(21) Anmeldenummer: 07115890.1
(22) Anmeldetag: 07.09.2007
(51) Int. Cl.: B65D 83/00, B05C 17/005

(54) **Kartuschenkolben**
Cartridge piston
Piston de cartouche

(30) Priorität: 06.10.2006 DE 102006047289
(43) Veröffentlichungstag der Anmeldung: 09.04.2008
(73) Patentinhaber: Sulzer Chemtech AG, 8404 Winterthur (CH)
(72) Erfinder: Brugner, Nikolaus, 86472, Ziemetshausen (DE)
(74) Vertreter: Sulzer Management AG

(56) Entgegenhaltungen:
- EP-A- 0 577 312
- EP-A- 1 514 812
- EP-B- 1 165 400
- DE-U1- 20 010 417
- DE-U1- 29 702 551
- US-A1- 2005 029 306

## Beschreibung

Die Erfindung betrifft einen Kartuschenkolben nach dem Oberbegriff des Anspruchs 1, wie aus DE 200 10417 U1 bekannt.

Weitere Kartuschenkolben sind aus der EP 1 165 400 B1 bekannt. Dort sind zwischen der Außenwand und einem inneren ringförmigen Stützkörper des Kartuschenkolbens sternförmig verlaufende radiale Verbindungsstege vorgesehen. Die Verbindungsstege weisen seitliche Rippen auf und verlaufen nahezu über die gesamte Höhe des Kartuschenkolbens. Wie besonders aus Figur 1 der EP 1 165 400 B 1 hervorgeht, sind die Verbindungsstege lediglich im Bereich einer auswärts geneigten Abstützlippe an der Unterseite des Kartuschenkolbens gegenüber der unteren Stirnseite geringfügig zurückversetzt, so dass sich eine stabile Verbindung zwischen dem inneren ringförmigen Stützkörper und der Außenwand ergibt. Dadurch sind derartige Kartuschenkolben relativ steif und können ohne größere Verformungen in Kartuschen eingesetzt und zum Ausdrücken der Kartuschen mit Hilfe von Eindrückstößeln verschoben werden. Weitere Beispiele für derartige Kolben sind aus der EP 1514 812 A1 bekannt.

Kartuschen mit derartigen Kartuschenkolben werden zum Austrag der in den Kartuschen befindlichen Materialien üblicherweise in speziell dafür vorgesehene Ausdrückpistolen mit einem auf den Kartuschenkolben wirkenden Eindrückstößel eingesetzt. Besonders bei kostengünstigen Ausdrückpistolen können die Eindrückstößel jedoch beim Eindrücken gegenüber der Kartuschenachse ausweichen, so dass die Kartuschenkolben einseitig bzw. schief eingedrückt werden. Dies kann bei steifen Kartuschenkolben zu Dichtproblemen führen.

Aufgabe der Erfindung ist es, einen Kartuschenkolben der eingangs genannten Art zu schaffen, der auch bei einseitiger bzw. schiefer Belastung durch einen Eindrückstößel ein gutes Abdichtverhalten gewährleistet.

Diese Aufgabe wird durch einen Kartuschenkolben mit den Merkmalen des Anspruchs 1 gelöst. Zweckmäßige Weiterbildungen und vorteilhafte Ausführungsformen der Erfindung sind Gegenstand der Unteransprüche.

Bei dem erfindungsgemäßen Kartuschenkolben ist zwischen der Außenwand und dem inneren Stützkörper des Kartuschenkolbens ein mit den Verbindungsstegen verbundener Versteifungskörper vorgesehen, wobei die Verbindungsstege an der Unterseite des Kartuschenkolbens zwischen der Außenwand und dem Versteifungskörper einen mindestens bis zur halben Höhe des Kartuschenkolbens reichenden Spalt aufweisen. Durch die den inneren Stützkörper und den mit diesem verrippten Versteifungskörper wird ein sehr steifer Kolbenkern geschaffen, der den Druck des Mediums ohne Deformation auf den Eindruckstößel übertragen kann. Die Außenwand des Kartuschenkolbens mit den Dicht- bzw. Abstützlippen ist aufgrund der Spalte in den Verbindungsstegen dagegen äußerst flexibel an dem Kolbenkern angelenkt, so dass sich die Dicht- und Abstützlippen auch dann flexibel an eine Kartuschenwand anpassen können, wenn die Kolbenauflage um einen Winkel geneigt ist. Bei einer z.B. durch Schiefstellung oder exzentrisches Drücken eines Eindrückstößels bedingten Schrägstellung des Kolbens kann sich der Kolbenkern neigen, während sich die Außenwand mit den Dicht- und Abstützlippen der Kartusche angleicht. Dadurch kann die Abdichtfunktion des Kartuschenkolbens selbst bei größeren Schiefstellungen oder einseitigen Belastungen des Eindrückstößels beibehalten werden.

In einer besonders zweckmäßigen Ausführung verjüngt sich der Spalt in den Verbindungsstegen von der Unterseite des Kartuschenkolbens nach oben hin keilförmig. Dadurch ist auch die Abstützlippe in radialer Richtung äußerst flexibel und kann radial stärker vorgespannt werden, ohne dabei große Reibungskräfte zu erzeugen.

In einer weiteren vorteilhaften Ausgestaltung weist der Kartuschenkolben auf seiner Oberseite eine nach oben offene randseitige Ringnut auf. Zwischen der Ringnut und dem Spalt in den Verbindungsstegen verbleibt somit nur noch ein relativ schmaler Steg zur Außenwand hin, so dass die Außenwand am stabilen Kartuschenkern in Art eines Drehgelenks und damit äußerst flexibel angebunden ist.

Radial innerhalb des in der Nähe der Außenwand angeordneten Versteifungskörpers können besonders bei größeren Kartuschenkolben auch einer oder mehrere weitere Versteifungskörper angeordnet sein.

Der innere Stützkörper kann in einer zweckmäßigen Ausführung hohlzylindrisch ausgeführt sein und ein Entlüftungsventil für die zwischen der Füllmasse und dem Kartsuchenkolben eingeschlossene Luft enthalten. Der innere Stützkörper kann aber auch als massiver Kern als Ausgangspunkt der zur Außenwand sternförmig verlaufenden radialen Verbindungsstege ausgeführt sein.

Weitere Besonderheiten und Vorzüge der Erfindung ergeben sich aus der folgenden Beschreibung bevorzugter Ausführungsbeispiele anhand der Zeichnungen. Es zeigen:
- Figur 1: einen erfindungsgemäßen Kartuschenkolben in einer Perspektive;
- Figur 2: den Kartuschenkolben von Figur 1 in einer Unteransicht;
- Figur 3: eine Schnittansicht entlang der Linien A-A von Figur 2;
- Figur 4: eine Schnittansicht entlang der Linien B-B von Figur 2;
- Figur 5: einen weiteren erfindungsgemäßen Kartuschenkolben in einer Unteransicht;
- Figur 6: eine Schnittansicht entlang der Linien A-A von Figur 5 und
- Figur 7: eine Schnittansicht entlang der Linien B-B von Figur 5.

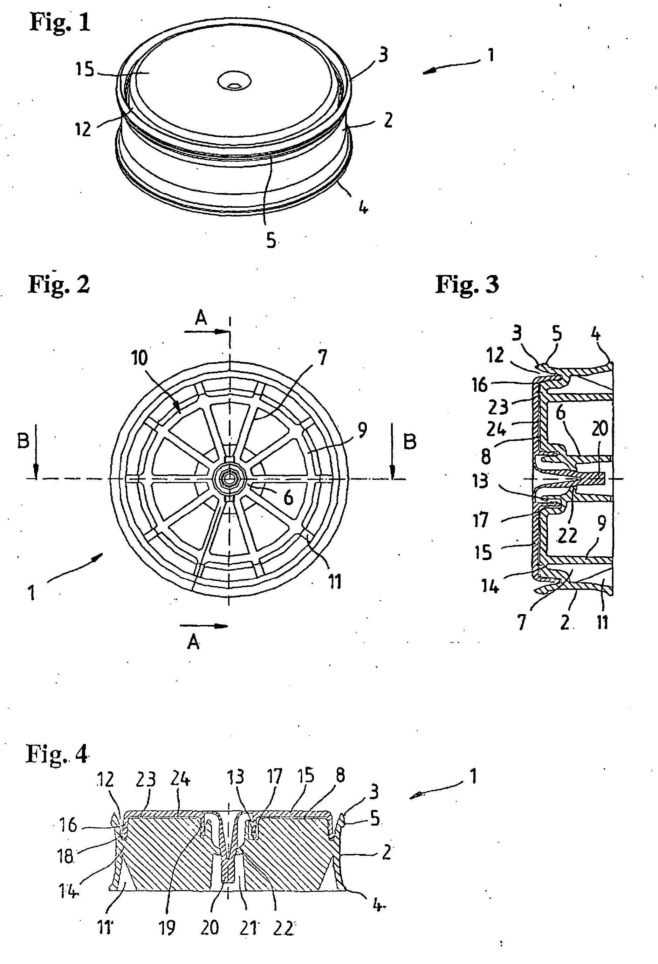

Der in den Figuren 1 bis 4 in unterschiedlichen Ansichten dargestellte Kartuschenkolben 1 ist als Ausdrückkolben für Kartuschen bestimmt, die zum Aufbewahren und Austragen unterschiedlichster Materialien, wie z.B. Klebstoffen, Dichtmitteln und ähnlichem, verwendet werden. Die Kartuschen werden zum Austragen in der Regel in handelsübliche Ausdrückpistolen eingesetzt. Diese Ausdrückpistolen enthalten einen Eindrückstößel, durch den der Kartuschenkolben 1 in die dazugehörige Kartusche eingedrückt und damit das in der Kartusche befindliche Material über eine Austragsöffnung der Kartusche ausgedrückt werden kann.

Der Kartuschenkolben 1 enthält eine Außenwand 2 mit einem radial nach außen vorstehenden oberen Rand 3 an der und einem ebenfalls radial nach außen vorstehenden unteren Rand 4 an der Unterseite. An dem oberen Rand 3 ist eine Dichtkante 5 angeformt. Durch den oberen Rand 3 mit der Dichtkante 5 wird eine Dichtlippe zur Abdichtung des Kartuschenkolbens 1 gegenüber der Innenwand einer Kartusche gebildet. Der untere Rand 4 bildet eine Abstützlippe zur Führung des Kartuschenkolbens 1 innerhalb der Kartusche. Der Außendurchmesser des unteren Rands 4 und des oberen Rands 3 mit der Dichtkante 5 ist größer als der Innendurchmesser der dazugehörigen Kartusche. Dadurch sind die Dichtlippe und die Abstützlippe vorgespannt.

Wie besonders aus den Figuren 2 und 3 hervorgeht, weist der Kartuschenkolben 1 radial innerhalb der Außenwand 2 einen zentralen inneren hohlzylindrischen Stützkörper 6 und radiale Verbindungsstege 7 auf, die ausgehend von dem hohlzylindrischen Stützkörper 6 sternförmig zu der Außenwand 2 verlaufen und die Außenwand 2 mit dem Stützkörper 6 verbinden. An der zum Kartuscheninhalt gewandten Oberseite enthält der Kartuschenkolben 2 eine in Figur 3 erkennbare Deckfläche 8. Zwischen dem inneren Stützkörper 6 und der Außenwand 2 ist ein mit den Verbindungsstegen 7 verbundener Versteifungskörper 9 vorgesehen. Der Versteifungskörper 9 ist bei der gezeigten Ausführung ringförmig und wird durch ringsegmentförmige Versteifungsstege 10 zwischen den Verbindungsstegen 7 gebildet. Die Versteifungsstege 10 können aber auch geradlinig verlaufen und der Versteifungskörper 9 kann eckig sein oder eine andere geeignete Form aufweisen.

Aus den Figuren 3 und 4 ist ersichtlich, dass die Verbindungsstege 7 an der Unterseite des Kartuschenkolbens 1 zwischen dem Versteifungskörper 9 und der Außenwand 2 einen bis zur Hälfte der Kolbenhöhe reichenden Spalt 11 aufweisen. Der Spalt 11 ist keilförmig ausgeführt und verjüngt sich in Richtung der Oberseite des Kartuschenkolbens 1. Die Unterseite des Versteifungskörpers 9 schließt mit Unterseite der Außenwand 2 bündig ab, so dass der Innenteil und der Außenteil etwa gleich hoch sind.

Der Kartuschenkolben 1 enthält an seiner zum Kartuscheninhalt gewandten Oberseite randseitig eine nach oben offene, im Querschnitt V-förmige äußere Ringnut 12 und eine dazu koaxiale innere Ringnut 13. Durch die äußere Ringnut 12 wird gewährleistet, dass der obere Rand 3 mit der Dichtlippe 5 seitlich flexibel ist und sich an die Innenwand einer Kartusche anlegen kann. Außerdem sorgt die äußere Ringnut 12 dafür, dass die durch den oberen Rand 3 mit der Dichtkante 5 gebildete Dichtlippe durch den beim Auspressen der Kartusche auf den Kartuschenkolben wirkenden Druck des Kartuscheninhalts radial nach außen an die Wand der Kartusche angedrückt wird. Der zwischen dem Spalt 11 und der oberen Ringnut 12 gemäß Figur 4 verbleibende Steg 14 ist relativ schmal, so dass die Außenwand 2 am stabilen Kartuschenkern in Art eines Drehgelenks und damit äußerst flexibel angebunden ist. Dadurch kann sich die Außenwand des Kartuschenkolbens optimal an die Innenwand der Kartusche anpassen.

Auf der Oberseite des Kartuschenkolbens 1 ist eine Abdeckscheibe 15 mit einem in die äußere Ringnut 12 eingreifenden, nach unten abgewinkelten äußeren Rand 16 und einem in die innere Ringnut 13 eingreifenden inneren Ringsteg 17 befestigt. An dem nach unten abgewinkelten äußeren Rand 16 und dem inneren Ringsteg 17 der Abdeckscheibe 15 sind in Figur 4 gezeigte Rastnasen 18 bzw. Rastnuten 19 vorgesehen, durch welche die Abdeckscheibe 15 über eine Rastverbindung am Kartuschenkolben 1 gehalten wird. Die Abdeckscheibe 15 besteht z.B. aus Polyamid oder einem anderen, gegenüber dem Kartuscheninhalt resistenten Material, um so den z.B. aus Polyethylen oder einem anderen weichen Kunststoff bestehenden Kartuschenkolben 2 zu schützen.

Wie in den Figuren 3 und 4 erkennbar, ist in der Mitte der Abdeckscheibe 15 ein nach unten ragender konischer Zapfen 20 axial beweglich angeformt. Der konische Zapfen 20 ragt in ein durchgehendes Loch 21 des inneren Stützkörpers 6 und bildet mit einer Ventillippe 22 ein Entlüftungsventil. Auf der Deckfläche 8 des Kartuschenkolbens 2 befinden sich unterbrochene Abstandsstege oder Abstandsnocken 23, durch welche die Abdeckscheibe 15 mit einem vorgegebenen geringen Spalt 24 von der Deckfläche 8 beabstandet gehalten wird. Der konische Zapfen 20 wird beim Einsetzen des Kartuschenkolbens 2 in die Kartusche durch einen entsprechenden Ansatz an einem Werkzeug von unten eingedrückt, so dass das Entlüftungsventil öffnet.

Dadurch kann in der Kartusche befindliche Luft über Zwischenräume zwischen den Rastnasen 18, den Spalt 24 zwischen der Deckfläche 8 und der Abdeckscheibe 15, einen Spalt zwischen der Ringnut 13 und dem Ringsteg 17 sowie das geöffnete Entlüftungsventil entweichen.

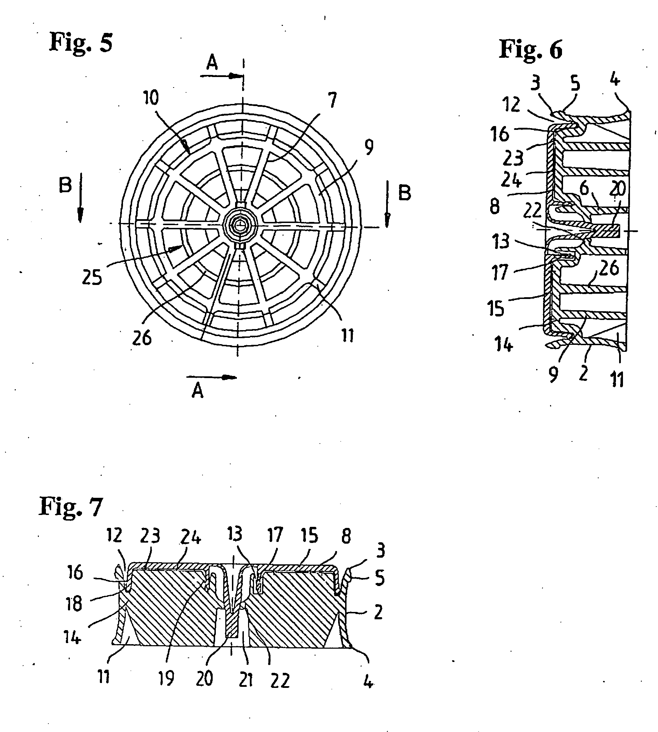

In den Figuren 5 bis 7 ist ein weiteres Ausführungsbeispiel eines Kartuschenkolbens gezeigt. Diese Ausführung unterscheidet sich von dem ersten Ausführungsbeispiel lediglich dadurch dass radial innerhalb des ringförmigen Versteifungskörpers 9 ein dazu konzentrischer weiterer ringförmiger Versteifungskörper 25 vorgesehen ist. Auch der Versteifungskörper 25 wird durch ringsegmentförmige Versteifungsstege 26 zwischen den sternförmig verlaufenden Verbindungsstegen gebildet. Ansonsten weist der Kartuschenkolben einen dem ersten Ausführungsbeispiel entsprechenden Aufbau auf, so dass einander entsprechende Bauteile auch mit denselben Bezugszeichen versehen sind.

## Patentansprüche

1. Kartuschenkolben (1) mit einer einem Kartuscheninhalt zuzuwendenden Oberseite, einer Unterseite, einer Außenwand (2), einem inneren Stützkörper (6) und radialen Verbindungsstegen (7) zwischen der Außenwand (2) und dem inneren Stützkörper (6), wobei zwischen der Außenwand (2) und dem inneren Stützkörper (6) ein mit den Verbindungsstegen (7) verbundener Versteifungskörper (9) angeordnet ist, wobei die Verbindungsstege (7) an der Unterseite des Kartuschenkolbens (1) zwischen der Außenwand (2) und dem Versteifungskörper (9) einen Spalt (11) aufweisen, **dadurch gekennzeichnet, dass** der Spalt (11) in den Verbindungsstegen (7) mindestens bis zur halben Höhe des Kartuschenkolbens (1) reicht.

2. Kartuschenkolben nach Anspruch 1, **dadurch gekennzeichnet, dass** sich der Spalt (11) in den Verbindungsstegen (7) von der Unterseite des Kartuschenkolbens (1) nach oben hin keilförmig verjüngt.

3. Kartuschenkolben nach Anspruch 1 oder 2, **dadurch gekennzeichnet, dass** der Kartuschenkolben (1) auf seiner Oberseite eine nach oben offene randseitige Ringnut (12) mit einem V-förmigen Querschnitt enthält.

4. Kartuschenkolben nach einem der vorhergehenden Ansprüche, **dadurch gekennzeichnet, dass** die Unterseite des Versteifungskörpers (9) mit der Unterseite der Außenwand (2) bündig abschließt.

5. Kartuschenkolben nach einem der vorhergehenden Ansprüche, **dadurch gekennzeichnet, dass** der Versteifungskörper (9) ringförmig ist.

6. Kartuschenkolben nach einem der vorhergehenden Ansprüche, **dadurch gekennzeichnet, dass** zwischen dem Versteifungskörper (9) und dem Stützkörper (6) mindestens ein weiterer Versteifungskörper (25) angeordnet ist.

7. Kartuschenkolben nach einem der vorhergehenden Ansprüche, **dadurch gekennzeichnet, dass** die Außenwand (2) einen radial nach außen vorstehenden oberen Rand (3) mit einer Dichtkante (5) und einen als Abstützlippe ausgebildeten, radial nach außen vorstehenden unteren Rand (4) enthält.

8. Kartuschenkolben nach einem der vorhergehenden Ansprüche, **dadurch gekennzeichnet, dass** auf der Oberseite des Kartuschenkolbens (1) eine Abdeckscheibe (15) angeordnet ist.

9. Kartuschenkolben nach Anspruch 8, **dadurch gekennzeichnet, dass** an der Abdeckscheibe (15) ein nach unten ragender konischer Zapfen (20) angeformt ist, der mit einer Ventillippe (22) im Stützkörper (6) ein Entlüftungsventil bildet.

10. Kartuschenkolben nach Anspruch 8 oder 9, **dadurch gekennzeichnet, dass** auf einer Deckfläche (8) des Kartuschenkolbens (1) unterbrochene Abstandsstege oder Abstandnocken (23) abgeordnet sind, durch welche die Abdeckscheibe (15) mit einem vorgegebenen geringen Spalt (24) von der Deckfläche (8) beabstandet gehalten wird.

## Claims

1. A cartridge piston (1) having an upper side extending towards a filling material and a lower side, an outer wall (2) an inner support body (6) and radial connection webs (7) between the outer wall (2) and the inner support body (6), wherein a stiffening body (9) connected to the connection webs (7) is arranged between the outer wall (2) and the inner support body (6); and in that the connection webs (7) have a gap (11) at the lower side of the cartridge piston (1) between the outer wall (2) and the stiffening body (9), **characterised in that** the gap (11) extends at least up to half the height of the cartridge piston (1) in the connection webs (7).

2. A cartridge piston in accordance with claim 1, **characterized in that** the gap (11) in the connection webs (7) converges upwardly in wedge shape from the lower side of the cartridge piston (1).

3. A cartridge piston in accordance with claim 1 or claim 2, **characterized in that** the cartridge piston (1) contains an upwardly open marginal ring groove (12) with a V-shaped cross-section at its upper side.

4. A cartridge piston in accordance with any one of the preceding claims, **characterized in that** the lower side of the stiffening body (9) terminates flush with the lower side of the outer wall (2).

5. A cartridge piston in accordance with any one of the preceding claims **characterized in that** the stiffening body (9) is annular.

6. A cartridge piston in accordance with any one of the preceding claims, **characterized in that** at least one further stiffening body (25) is arranged between the stiffening body (9) and the support body (6).

7. A cartridge piston in accordance with any one of the preceding claims, **characterized in that** the outer wall (2) contains a radially outwardly projecting upper rim (3) with a sealing edge (5) and a radially outwardly projecting lower rim (4) configured as a support lip.

8. A cartridge piston in accordance with any one of the preceding claims **characterized in that** a cover plate (15) is arranged on the upper side of the cartridge piston (1).

9. A cartridge piston in accordance with claim 8, **characterized in that** a conical spigot (20) is formed on the cover plate (15) and projects downwardly and forms a bleeding valve with a valve lip (22) in the support body (6).

10. A cartridge piston in accordance with claim 8 or claim 9, **characterized in that** interrupted spacing webs or spacing cams (23) are arranged at a cover surface (8) of the cartridge piston (1) and the cover plate (15) is held by them spaced apart from the cover surface (8) with a predetermined small gap (24).

## Revendications

1. Piston de cartouche (1) avec un côté supérieur à orienter vers un contenu de cartouche, un côté inférieur, une paroi extérieure (2), un corps de support intérieur (6) et des nervures de liaison radiales (7) entre la paroi extérieure (2) et le corps de support intérieur (6), où est disposé entre la paroi extérieure (2) et le corps de support intérieur (6) un corps de renforcement (9) relié aux nervures de liaison (7), où les nervures de liaison (7) présentent au côté inférieur du piston de cartouche (1) entre la paroi extérieure (2) et le corps de renforcement (9) une fente (11), **caractérisé en ce que** la fente (11) dans les nervures de liaison (7) s'étend au moins jusqu'à mi-hauteur du piston de cartouche (1).

2. Piston de cartouche selon la revendication 1, **caractérisé en ce que** la fente (11) dans les nervures de liaison (7) retrécit en forme de coin depuis le côté inférieur du piston de cartouche (1) vers le haut.

3. Piston de cartouche selon la revendication 1 ou 2, **caractérisé en ce que** le piston de cartouche (1) présente à son côté supérieur une rainure annulaire (12) côté bord, ouverte vers le haut avec une section transversale en forme de V.

4. Piston de cartouche selon l'une des revendications précédentes, **caractérisé en ce que** le côté inférieur du corps de renforcement (9) est en affleurement avec le côté inférieur de la paroi extérieure (2).

5. Piston de cartouche selon l'une des revendications précédentes, **caractérisé en ce que** le corps de renforcement (9) est annulaire.

6. Piston de cartouche selon l'une des revendications précédentes, **caractérisé en ce qu'**il est disposé entre le corps de renforcement (9) et le corps de support (6) au moins un autre corps de renforcement (25).

7. Piston de cartouche selon l'une des revendications précédentes, **caractérisé en ce que** la paroi extérieure (2) comprend un bord supérieur (3) faisant saillie radialement vers l'extérieur avec une arête d'étanchéité (5) et un bord inférieur (4) faisant saillie radialement vers l'extérieur, réalisé comme lèvre d'appui.

8. Piston de cartouche selon l'une des revendications précédentes, **caractérisé en ce qu'**un disque de recouvrement (15) est disposé au côté supérieur du piston de cartouche (1).

9. Piston de cartouche selon la revendication 8, **caractérisé en ce qu'**est rapporté par formage au disque de recouvrement (15) un pivot conique (20) faisant saillie vers le bas qui forme avec une lèvre de vanne (22) dans le corps d'appui (6) une vanne d'aération.

10. Piston de cartouche selon la revendication 8 ou 9, **caractérisé en ce que** sont disposés sur une face de recouvrement (8) du piston de cartouche (1) des nervures d'écartement ou saillies d'écartement interrompues (23) par lesquelles le disque de recouvrement (15) est maintenu à distance de la face de recouvrement (8) selon une petite fente prédéterminée (24).
